# EUROPEAN PATENT APPLICATION

(11) **EP 3 653 237 A1**
(43) Date of publication of application: **20.05.2020**
(21) Application number: 18832169.9
(22) Date of filing: 09.07.2018
(51) Int. Cl.: A61M 1/04, A61M 27/00, A61M 39/24, A61M 39/20

(54) **MEDIASTINAL DRAINAGE SYSTEM**

(30) Priority: 11.07.2017 BR 102017014889
(71) Applicant: Zammi Instrumental Ltda, 25251-300 Duque de Caxias - RJ (BR)
(72) Inventor: VARGAS FONSECA, Luiz Henrique, 25251-300 Duque De Caxias (BR)
(74) Representative: Weickmann & Weickmann PartmbB
(86) International application number: PCT/BR2018/050231
(87) International publication number: WO 2019/010555

(57) **Abstract**

The present invention relates to a system comprising a flexible bag for storing the drained secretion with an anti-collapse device (9), being connected on one side to a "T" valve which is provided with protective lid and, on the other side, to the flow control backflow preventer valve consisting of a one-piece circular core (2) of elastomeric material which is secured to the housing, said core containing a central plug connected by radial rods to the borderline of the circular body thereof, so that said core is used in two-way housings, an inlet and an outlet, said valve composed of a one-way valve. Said system further comprising within the housing a clot fractioning device at the inlet and an anti-clogging device from the maximum opening point of the core (2).

## Description

### FIELD OF THE INVENTION

The present invention relates to a mediastinal or pleural drainage collection system for removing air, liquid and solid (e.g., clot) from the mediastinal or pleural space of the patient, which can result from infectious processes, trauma, surgical procedures, among others.

### BACKGROUND OF THE INVENTION

The mediastinal drainage collector is a hospital-medical device used in thoracic surgeries and/or heart surgeries and is intended to drain the liquid and/or gaseous content from the thoracic cavity. In intensive care units, chest drainage and thoracentesis are frequent procedures, respectively, in the treatment and diagnosis of pleural complications.

These devices, in addition to being designed for fluid drainage, enable the control by measuring the amount and type of drainage secretion from pleural or pericardial effusions, empyema, hemothorax, pneumothorax. The mediastinal or pleural drainage collection system use the theory of underwater siphoning, theory based in a one-way valve, also called closed pleural drainage or water sealed drainage.

The underwater siphoning or water seal prevents the open pneumothorax from occurring, that is, the entry of atmospheric air into the thoracic cavity (pleural, pericardial or mediastinal cavity), maintaining the intrathoracic pressure in balance, which is negative in relation to the atmospheric pressure.

### BRIEF DESCRIPTION OF THE FIGURES

The present invention may be well understood from the accompanying illustrative figures, which represent it in a schematic and non-limiting manner:
- Figure 1 - Schematic diagram of a mediastinal drainage device of the state of the art;
- Figure 2 - Schematic diagram of a suction drainage system of the state of the art;
- Figure 3 - Schematic diagram of collector system without immersed rod or liquid in the collecting bottle of the state of the art;

- Figure 4 - Schematic diagram of collector system with three bottles of the state of the art;
- Figure 5 - Schematic diagram of drainage system of the state of the art;
- Figure 6 - Illustration of alternative devices of the state of the art;
- Figure 7 - Schematic diagram of the backflow preventer valve used in the present invention;
- Figure 8 - Schematic diagram of the system of the present invention;
- Figure 9 - Schematic diagram of the anti-collapse device according to the present invention;
- Figure 10 - Backflow preventer valve used in the present invention adapted to provide an automatic opening air outlet;
- Figures 11 A-C - Schematic diagram of the operation of the backflow preventer valve used in the present invention;
- Figure 12 - Schematic diagram of the drainage system with a backflow preventer valve (23 or 24) adapted to the air outlet;
- Figure 13 - Schematic diagram of the improved backflow preventer valve used in the present invention;
- Figure 14 - Schematic diagram of the valve, detailing the clot fractioning device in the valve inlet connector and the anti-clogging device in the valve outlet connector;
- Figure 15 - Schematic diagram of the improved backflow preventer valve of the present invention, illustrating vigorous milking situation, wherein the anti-clogging device acts to prevent the displacement of the valve beyond the maximum opening position.

### STATE OF THE ART

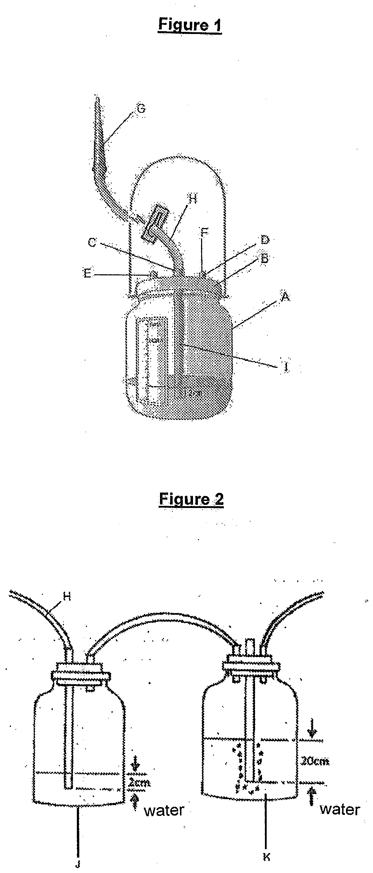

Figure 1 illustrates a system of the state of the art consisting of a rigid bottle (A) provided with lid (B) having two connectors (C, D), an air inlet (E) and a breather tube (F). One of these connectors is connected to the chest tube (G) through the tube (H) and the other is open to the atmosphere. For the system to work, the bottle should have a water seal or saline (I) until the level is at least 2 cm above the breather tube outlet. To this arrangement, the chest tube is added which after being inserted into the thoracic or pleural cavity is connected to the arrangement by the drainage tube.

The water seal or siphoning acts as a one-way content outflow valve efferent to the pleura wherein the fluids or gases housed in the cavity are removed by gravity. The flow is established from the upper compartment to the lower one, the rigid collecting bottle must be kept lower than the patient's chest in order that pneumothorax may not occur.

Although this device is widely used in medical practice, there are some issues related thereto, such as the bottle must have to be at a level below the patient's pleural or mediastinal cavity, otherwise there is no drainage. Also, siphoning of the bottle contents to the patient's chest cavity is likely to occur, leading to serious consequences.

The bottle should be kept in an upright position with the outlet hole of the tube submerged in the water seal liquid, otherwise air may enter the patient's chest cavity and cause a pneumothorax.

To allow drainage, the bottle lid must have an air inlet, the bottle contents are thus in contact with ambient air and are subject to contamination. In addition, due to the internal pressure of the patient's chest cavity, a column of fluid forms in the drainage tube, which oscillates up and down along with the patient's breathing. Hence, if contents of the bottle are contaminated, the contamination of the drainage tube and, consequently, the patient, may occur.

The water seal acts as a "one-way" valve, preventing air from entering the chest cavity, but not the return of fluid through the tube. For functioning, the liquid level must be at least 2 cm above the outlet hole of the gas outflow breather tube. Therefore, in order for drainage to occur, the pressure within the cavity should be greater than the liquid column's above the tube outlet hole. As drainage occurs, the level increases, complicating the drainage even more.

To minimize this effect, healthcare professionals often empty the bottle periodically and change the inside fluid. However, such a procedure, in addition to generate additional work, provides a great risk to the patient, because if inaccurately done, it can result in various problems, such as pneumothorax, return of the drained liquid, bleeding and contamination, and it must be done whenever the level raises, generating the need for constant vigilance by the professional. Furthermore, due to the water seal, the normal pressure in the chest cavity, which should be between -4mmHg and -8mmHg, never reaches these values, since due to the liquid column at the outlet of the water seal tube, the drainage ceases while the pressure is still positive. Therefore, the drainage is not complete as it is interrupted by the still positive pressure in the cavity.

Due to all the problems, water seal drainage needs constant care and attention, which forces the patient to be hospitalized during the procedure. Additionally, this device considerably hinders the mobilization of the patient, preventing them from leaving the hospital.

As an alternative to accelerate drainage, it is common to use a procedure known as milking, in which the healthcare professional, with the aid of a forceps, compresses and slides the forceps along the drainage tube from the patient to the bottle, temporarily generating a negative pressure. However, this procedure, besides demanding a lot of physical effort, is extremely inefficient, since as soon as the forceps is opened, the pressure in the system equalizes with the ambient pressure and the negative pressure ceases.

Moreover, in order to minimize the effects of the water seal liquid column and improve the anti-backflow effect, up to three bottles can be used for a suction drainage system.

Figure 2 illustrates a set of two bottle (J, K), and one of them will necessarily serve as a one-way valve, while the second bottle will monitor the amount of negative pressure applied to the pleural space by a continuous suction source that depends on the difference of the liquid column between the two bottles.

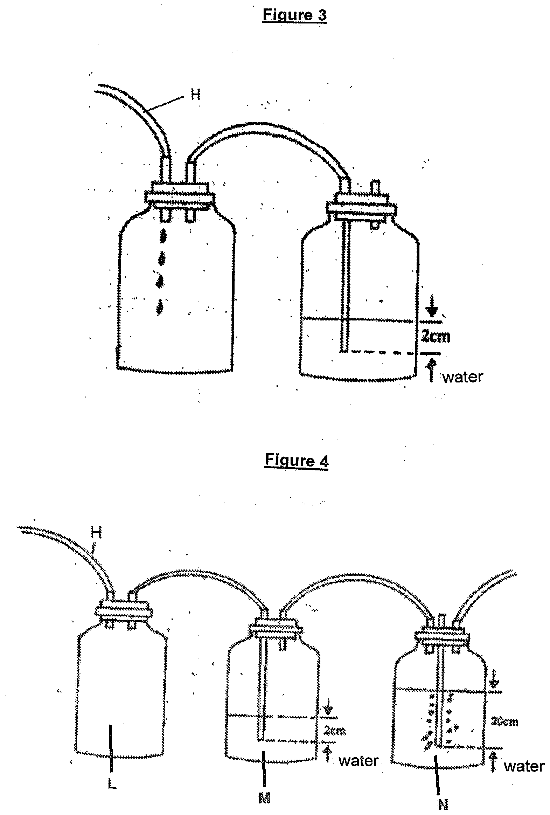

Figure 3 shows another type of collector device connected to the pleural drain without submerged or liquid rod in the collecting bottle, used for collecting blood for the purpose of autotransfusion in continuous traumatic bleeding situations.

As the two-bottle system, the three-bottle system (L, M, N) illustrated in Figure 4 requires a continuous suction generating source (not shown). The first collecting bottle (L) does not interfere with the suction drainage. The second bottle (M) acts as a one-way valve and the third bottle (N) controls the suction exerted on the system. The suction generating source will be conditioned upon the depth difference of the submerged water seal rods.

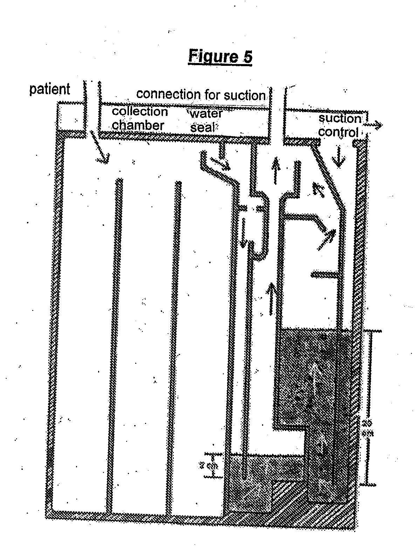

There are also more modern drainage systems wherein the drainage bottles are replaced by a single system comprising a collection chamber, water seal chamber and suction control chamber, as shown in Figure 5. However, although more sophisticated, the principle of operation and the occurrence of technical problems and patient exposure to infections remain the same as that of the other devices described above.

Thus, it has been observed that the dual or multiple bottle models, for example, those of Figures 2, 3 and 4, solve some of the problems of the single bottle model, but produce other problems related to the complexity of the procedure, cost increase, patient ambulation restriction and risks related to the use of vacuum network, such as pressure variation and loss, among others. The model illustrated in Figure 5, in addition to being costly, is also subject to the same problems, such as complexity of the procedure, cost increase, patient ambulation restriction and risks related to the use of vacuum network, such as pressure variation and loss, among others.

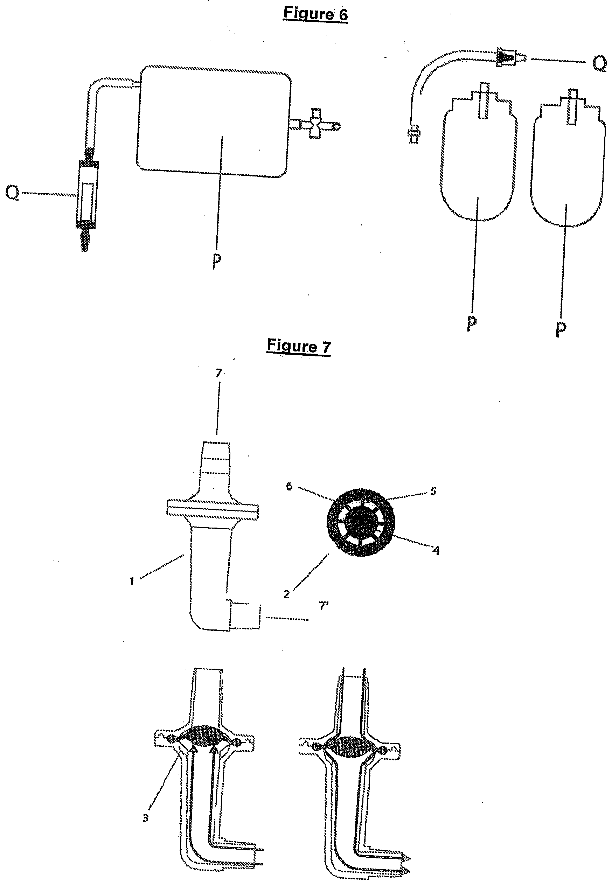

Figure 6 illustrates devices attempting to solve some of these problems by using flexible bags (P) instead of rigid bottles (A), and backflow preventer valves (Q) instead of water seal (I). However, these devices also have some problems that will be described below.

One of the problems with these devices is that the backflow preventer valve for use in drainage systems must have low opening pressure and zero backflow, especially at low pressures. These devices use duckbill-type backflow preventer valves. With this type of valve, it is practically impossible to achieve both of these characteristics at the same time (low opening pressure and zero backflow), and one of them should be chosen in the design specifications. In the devices shown in Figures 1-5, the low backflow feature at low pressures had been chosen. Therefore, these valves have a high opening pressure when compared to the water seal. Thus, while allowing greater patient mobility, these devices prolong treatment, requiring the patient to remain longer with the drain. Another common problem with this type of device is the blood accumulation around the valve, with the consequent coagulation thereof, which can lead to problems such as contamination, clogging and loss of valve efficiency. In addition, clots from the patient may accumulate inside the valve, which may prevent the valve from closing completely, causing it to allow backflow and air intake.

To overcome the problem of high opening pressure, vacuum suction could be used, but as these systems use flexible bags, this is not possible. Another feature would be milking, but these valves are not efficient enough to maintain the vacuum, and just like in the water seal, the negative pressure generated by milking is only temporary.

Another problem presented by these devices relates to high cost when compared to rigid bottles. In addition, these devices are generally designed for use in pneumothorax or hemothorax drainage procedures and cannot be used in both procedures at the same time, which represents a limitation of use. This is explained by the fact that it is common for a patient with hemothorax, for example, to develop into a pneumothorax, or a patient with pneumothorax to have occasional bleeding. This limits the use of such devices that use closed bags that have no air outlet required in cases of pneumothorax. Also, in devices developed for pneumothorax, the reservoirs are too small in case of bleeding, making their use unfeasible.

In addition, flexible bags may collapse or clog the bag inlet tube, depending on placement, impairing drainage to be performed.

These observations led to the need for producing a system that solves definitely, or at least drastically minimizes, the problems presented by the devices of the state of the art described above. Therefore, a continuous action system that is independent of the external vacuum network, does not restrict patient mobility and provides a lower risk of infections without excessively burdening the procedure is highly desired by those in the field.

The present invention precisely proposes the correction of the problems described above for the drainage devices of the state of the art.

### DESCRIPTION OF THE INVENTION

The present invention therefore relates to a mediastinal drainage collection system comprising a flexible bag and a Cartwheel-type flow control backflow preventer valve, such as that object of the patent application WO201502481 belonging to the same Applicant. Figures 7-15 illustrate the elements of the system according to the present invention.

Figure 7 schematically illustrates the flow control backflow preventer valve (1) having features that make it ideal for use in the mediastinal drainage system according to the present invention. Said valve (1) comprises a one-piece circular core (2) of elastomeric material which is secured to the housing (3) or body (3) of the valve (1). Said core containing a central plug (4) connected by radial rods (5) to the borderline (6) of the circular body thereof. Thus, said core (2) is used in two-way housings, an inlet (7) and an outlet (7'), said valve (1) composed of a one-way valve.

Moreover, said valve (1) has the advantages of allowing a very low opening pressure, having zero backflow, no areas of stagnation that can allow blood accumulation and coagulation, and reduced size and internal volume.

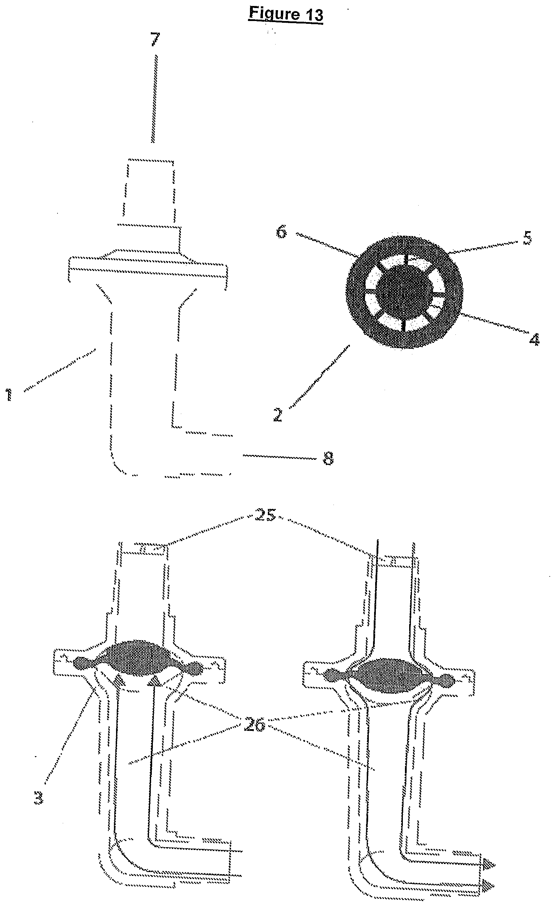

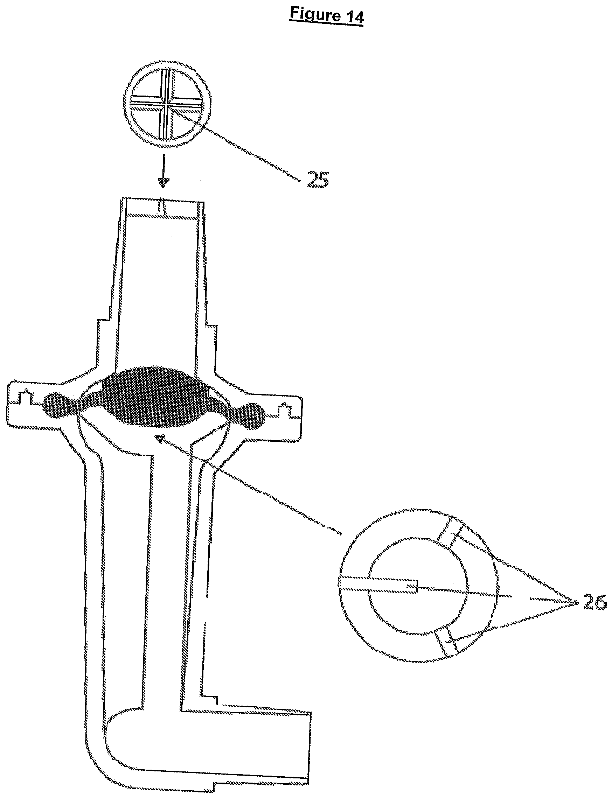

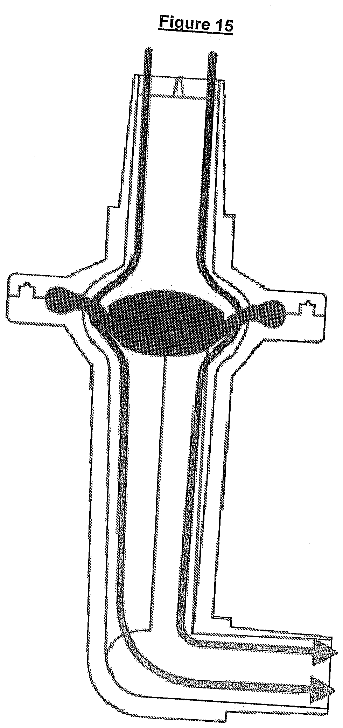

The housing (3) of the valve (1) has been further refined to allow better operation of the drainage function. One of the improvements was the inclusion of a clot fractioning device (25) in the inlet (7) of the housing (3) of the valve (1). This clot fractioning device (25) is composed of cross-shaped blades as shown in Figure 13 and in greater detail in Figure 14, where there is also a top view illustration of the clot fractioning device (25). These blades cut out possible clots coming from the patient, which could eventually get caught between the radial rods (5) of the core (2) and impair the valve operation (1). With this improvement, the chance of malfunction due to clots coming from the patient is greatly reduced. Another improvement is the anti-clogging device (26) within the valve housing (3) or body as shown in Figure 13 and in greater detail in Figure 14, said being formed by blades starting from the maximum opening point of the core (2) towards the outlet flow (7'), and creates a bulkhead that prevents the displacement of the core (2) beyond the position of its maximum opening, which could occur in the case of vigorous milking of the tube as shown in Figure 15. Therefore, the anti-clogging device (26) ensures the outflow of the valve (1) rendering it much safer. Still, Figure 14 illustrates the top view of the anti-clogging device (26).

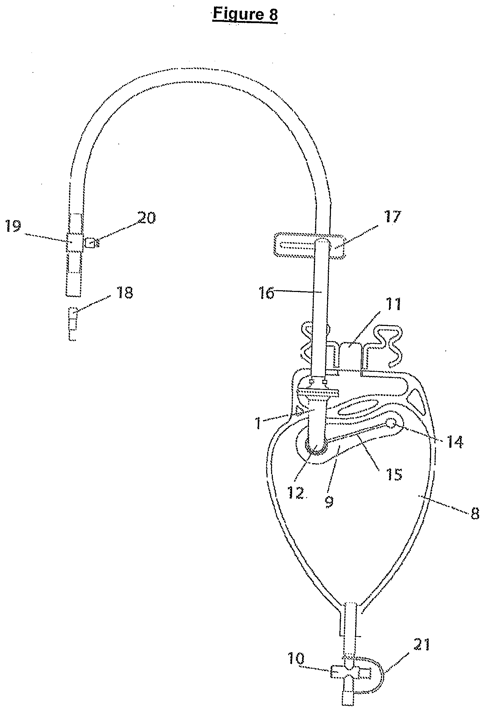

Figure 8 illustrates the complete drainage system wherein a flexible bag (8) for storing the drained secretion which has an anti-collapse device (9) and is connected to a "T" valve (10), or the like, used for disposal of the bag contents, which is provided with a protective lid (21) and, on the other side, the Cartwheel-type high-efficiency backflow preventer valve (1), can be seen. Said flexible bag (8) also has a handle (11) for fixing and transport, specially developed to confer greater flexibility and feasibility on the drainage system.

The anti-collapse device (9) has an inlet connector (12) and a channel system (13) that prevents collapsing of the flexible bag (8) and obstruction of the valve outlet (1). It also has an air outlet having a female luer connector (14) which can also be used as a sampling point and a protective lid (15) for the female luer connector.

Attached to the high-efficiency backflow preventer valve (1) is a drainage tube (16) made of flexible material so as to provide more convenience and comfort to the patient, and to enable the milking procedure with occlusion forceps (17). A scaled tapered connector (18) provides connection of the drainage system according to the present invention with drains of any size and a "T" connector (19) with female luer outlet with a needleless valve (20) for syringe access allows suction and flushing of the drainage tube (16) in case of clot obstruction.

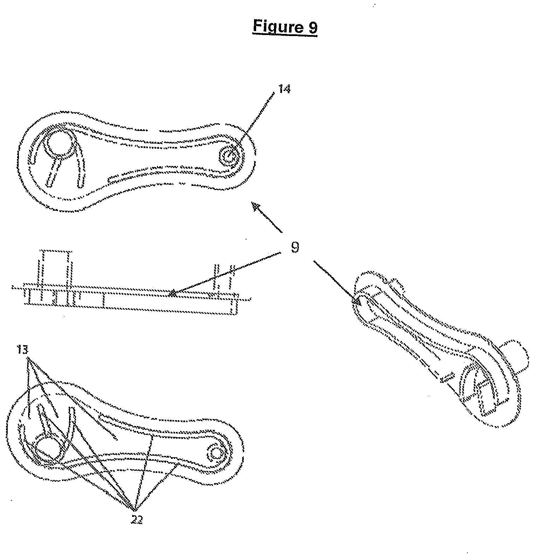

Among all the improvements introduced by the drainage system according to the present invention, special emphasis should be given to said anti-collapse device (9), as specifically illustrated in Figure 9.

According to said Figure 9, the anti-collapse device (9) has a standard female luer air outlet (14), with protective lid (15), which can be used to allow air outflow when the patient presents air leakage, or even allow the suction of the contents of the flexible bag (8) with a syringe to collect samples. Said anti-collapse device (9) also has a channel system (13) formed by vertical walls (22) that rise from the base of the device to ensure a spacing between the rear face of the flexible bag (8) and the inlet tube (16) shown in Figure 8, such that the flexible bag (8) will never collapse in the region covered by said anti-collapse device (9), and it will also ensure that the inlet connector section (12), connected to the outlet of the high-efficiency backflow preventer valve (1) is never obstructed, regardless of its positioning. Furthermore, said anti-collapse device (9) ensures a clear path for the air outflow from the flexible bag (8).

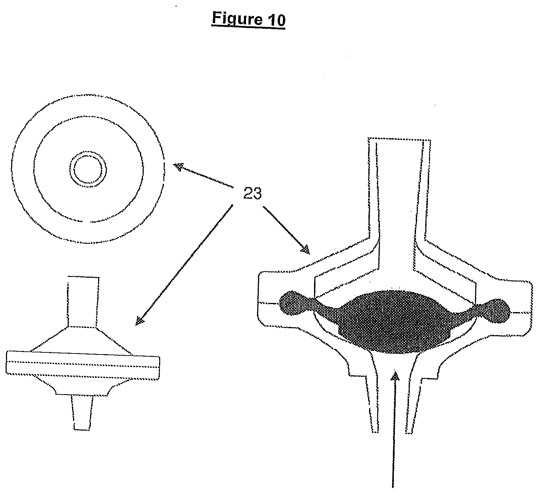

It should be noted that, optionally, the anti-collapse device (9) may receive at its standard luer air outlet (14) a second valve (23) similar to that of a backflow preventer valve (1) adapted to provide an automatic opening air outlet as shown in Figure 10.

This valve (23) can be fitted directly into the air outlet (14) of the anti-collapse device (9). Said valve (23) keeps the air outlet closed when there is no pressure inside the flexible bag (8). If air leaks and the air pressure inside the flexible bag (8) increases, said valve (23) opens automatically, allowing the air outflow and relieving internal pressure so as to allow drainage. Likewise, being a backflow preventer valve, said valve (23) prevents air from entering the flexible bag (8), keeping the drained contents sterile and free of contamination.

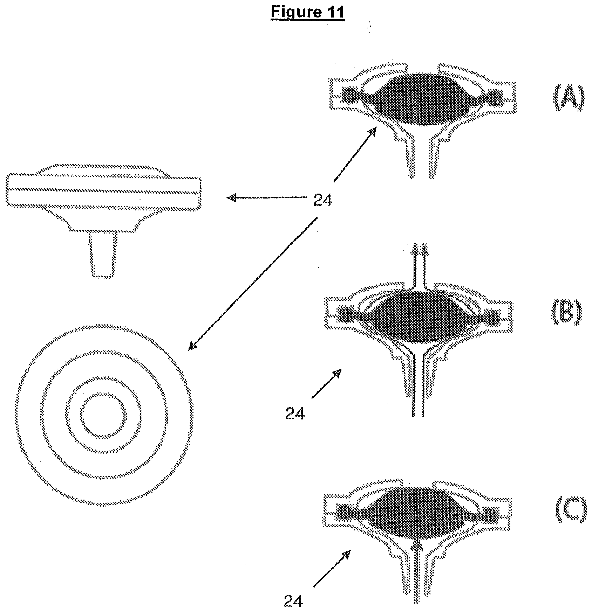

In addition, another version of said valve (23), the valve (24) as seen in Figure 11, in the drainage system application according to the present invention, has a special feature regarding its closure to prevent the bag contents from overflowing in situations that normally occur when the pressure inside the flexible bag (8) drastically increases, or a very fast flow occurs. These occurrences usually manifest when the flexible bag is full and for some reason is compressed, which would lead to leakage of its drained contents. That is, the valve (24) would allow air outflow, but would not allow the outflow of the liquid contained within the flexible bag (8). This special feature is possible due to the different design of the internal valve compartment (24). It is noteworthy that the top of the valve plug is very close to the valve outlet, so that the spacing between the plug and the outlet is sufficient for air outflow, as shown in Figure 11-B, but in case of increased liquid flow, the valve rises and closes the outlet as shown in Figure 11-C, preventing accidental leakage of the bag contents. In the case of the other valve (23), the internal valve compartment design is similar to that of the main valve (1) and is designed to ensure that there is no outflow obstruction under any circumstances, while also allowing high air flow therethrough. Thus, this valve (23) does not prevent accidental leakage of the bag contents and, therefore, when used, requires the bag to be at the upright position in order to prevent leakage of the bag contents. However, this valve (23) has a standard female luer outlet connector, which can be closed with the protective lid (15) so as to prevent leakage of the bag contents in case of patient transport, for example.

Figures 11A-C illustrate a schematic diagram of the aforementioned situations with respect to valve (24). Figure 11-A shows the valve (24) at rest, i.e. in the closed position. Figure 11-B shows the valve (24) in the open position for air passage, and Figure 11-C shows the valve (24) in the closed position due to the high inlet pressure, preventing the liquid outflow.

This feature of the mediastinal drainage system according to the present invention is especially interesting for home care treatment, as it reduces the need for checking and maintaining the flexible bag (8), ensuring drainage even in the event of air leakage, and protecting the patient against accidental spillage of the contents of the flexible bag (8). In addition, since the valve (23 or 24) keeps the system closed and the contents of the flexible pouch (8) isolated from ambient air, in the case of hospital treatment, blood collected in the early postoperative hours can be reinfused into the patient itself.

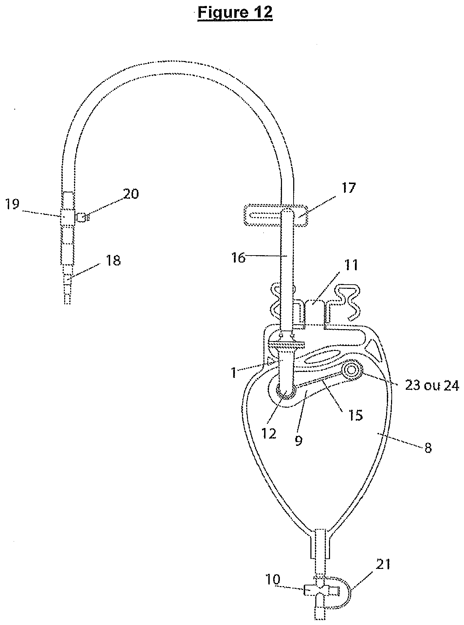

It can also be seen in Figure 12 the complete system with the adapted backflow preventer valve (23 or 24) installed at the air outlet of the anti-collapse device.

By the above description, a person skilled in the art involving mediastinal or pleural drainage will appreciate that the system developed in accordance with the present invention will provide, among others, the following immediate advantages:
- have a system including an extremely efficient valve that ensures drainage of all secretion from the patient's chest cavity;
- have a fully portable system that allows patient transport safely and without special care, and can even be carried on a stretcher;
- have a maintenance-free system;
- have a system that has air outlet in case of air leakage, with optional backflow preventer valve, which keeps the contents of the sterile flexible bag free of contamination and prevents accidental spillage of the contents of the flexible bag;
- have a system that allows the collection of secretion samples for laboratory analysis;
- have a system that enables the contents of the flexible bag to be drained without endangering the patient;
- have a system that allows the replacement of the flexible bag, if necessary, simply by disconnecting the connector and connecting it to a new flexible bag;
- have a system that, due to the efficiency of the valve, allows the generation of negative pressure within the patient's chest cavity, with a light milking procedure;
- have a system that allows connection to common chest drains of any size and the possibility of suctioning or flushing the drainage tube in the closed system in case of clot obstruction using only a needleless syringe through the valve installed in the extension of the drainage pipe;
- have a clot fractioning device (25) at the inlet (7) of the housing (3) of the valve (1), preventing valve malfunction due to clots coming from the patient;
- have an anti-clogging device (26) within the housing (3) of the valve (1) composed of longitudinal blades starting from the maximum opening point of the core (2) towards the outlet flow (7'), preventing the displacement of the valve plug beyond the maximum opening position and, consequently, its rupture in case of vigorous milking of the drainage tube.

## Claims

1. Mediastinal drainage system **characterized in that** it comprises a flexible bag (8) for storing the drained secretion with an with an anti-collapse device (9), being connected on one side to a "T" valve (10) which is provided with protective lid (21) and, on the other side, to the flow control backflow preventer valve (1) consisting of a one-piece circular core (2) of elastomeric material which is secured to the housing (3), said core containing a central plug (4) connected by radial rods (5) to the borderline (6) of the circular body thereof, so that said core (2) is used in two-way housings, an inlet (7) and an outlet (7'), said valve (1) composed of a one-way valve.

2. Mediastinal drainage system according to claim 1, **characterized in that** said flexible bag (8) also has a handle (11) for fixing and transport.

3. Mediastinal drainage system according to claim 1, **characterized in that** said anti-collapse device (9) is provided with an inlet connector (12) and a channel system (13) which prevents collapsing of the flexible bag (8) and clogging of the valve outlet (1), further having an air outlet with female luer connector (14) and a protective lid (15) for said female luer connector.

4. Mediastinal drainage system according to claim 1, **characterized in that**, connected to said backflow preventer valve (1), there is a drainage tube (16) made of flexible material to allow the procedure of milking with occlusion forceps (17).

5. Mediastinal drainage system according to claim 1, **characterized in that** it further comprises a scaled tapered connector (18) for connecting said drainage system with drains of any size and a "T" connector (19) with female luer outlet provided with a needleless valve (20) for syringe access, thus allowing suction and flushing of the drainage tube (16).

6. Mediastinal drainage system according to claim 1, **characterized in that** said anti-collapse device (9) can receive at its standard luer air outlet (14) a second valve (23) similar backflow preventer valve (1), adapted to provide an automatic opening air outlet.

7. Mediastinal drainage system according to claim 1, **characterized in that** it may alternatively include another anti-leakage valve (24) installed at the air outlet of the anti-collapse device (9), in which the top of the plug positioned nearby the valve outlet.

8. Mediastinal drainage system according to claim 1, **characterized in that** the inlet (7) of the housing (3) is provided with a clot fractioning device (25) composed of cross-shaped blades.

9. Mediastinal drainage system according to claim 1, **characterized in that** the housing (3) is provided with an anti-clogging device (26) composed of longitudinal blades from the maximum opening point of the core (2).
